Europäisches Patentamt

European Patent Office

Office européen des brevets

(19)

(11) **EP 1 609 475 A1**

(12) **EUROPEAN PATENT APPLICATION**
published in accordance with Art. 158(3) EPC

(43) Date of publication:
**28.12.2005 Bulletin 2005/52**

(21) Application number: **04725517.9**

(22) Date of filing: **02.04.2004**

(51) Int Cl.7: **A61K 33/44**, A61P 19/08,
A61P 13/12

(86) International application number:
**PCT/JP2004/004839**

(87) International publication number:
**WO 2004/089384 (21.10.2004 Gazette 2004/43)**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **03.04.2003 JP 2003100272**

(71) Applicant: **KUREHA CHEMICAL INDUSTRY CO.,
LTD.**
**Tokyo 103-8552 (JP)**

(72) Inventor: **YAMATO, Hideyuki,
1210813 (JP)**

(74) Representative: **HOFFMANN EITLE
Patent- und Rechtsanwälte
Arabellastrasse 4
81925 München (DE)**

(54) **REMEDY OR PREVENTIVE FOR LOW TURNOVER BONE DISEASES**

(57) An agent for treating or preventing low-turnover bone diseases, comprising as an active ingredient a spherical activated carbon, is disclosed. The low-turnover bone diseases include renal osteodystrophy and adynamic bone disease, caused by a low-turnover bone. The adynamic bone disease includes adynamic bone disease in conservative chronic kidney disease, and renal osteodystrophy in a patient under treatment with dialysis.

EP 1 609 475 A1

**Description**

TECHNICAL FIELD

This invention relates to an agent for treating or preventing diseases caused by a low-turnover bone (i.e., low-turnover bone diseases) containing a spherical activated carbon as an active ingredient.

BACKGROUND ART

Chronic kidney disease, particularly bone abnormalities involved in a hemodialysis, is known as renal osteodystrophy (ROD). Chronic kidney disease is recognized as a pathosis caused by a high bone turnover in which osteogenesis and bone resorption are active, such as osteitis fibrosa caused by secondary hyperparathyroidism (2HPT) or osteomalacia caused by an accumulation of aluminum, and therapeutic agents, treatments and preventions therefor have been developed.

However, it was recently reported that cases of chronic kidney disease caused by a low-turnover bone were increasing, particularly renal osteodystrophy involved in a hemodialysis, and it is known that when such a pathosis progresses, adynamic bone disease will develop (non-patent references 1 and 2). It has been revealed that renal osteodystrophy includes various diseases caused by different mechanisms, such as renal osteodystrophy caused by a low-turnover bone or renal osteodystrophy caused by a high bone turnover.

The adynamic bone disease and renal osteodystrophy caused by a low-turnover bone are pathophysiologically unknown, and therapeutic agents, treatments and preventions therefor are desired.

The present inventor has engaged in pathophysiological research on various diseases caused by a low-turnover bone and in development of treatments therefor and, as described below, completed the expression of a pathosis of low-turnover bone diseases in an animal. The invention was filed as Japanese patent application No. 2002-311101.

(non-patent reference 1) Ogata et al., "CLINICAL CALCIUM", (Japan), 2001, Vol. 11, No. 8, p. 999-1004
(non-patent reference 2) Yokoyama et al., "CLINICAL CALCIUM", (Japan), 2001, Vol. 11, No. 8, p. 1005-1013

DISCLOSURE OF INVENTION

An object of the present invention is to provide an agent for treating or preventing diseases caused by a low-turnover bone, i.e., low-turnover bone diseases.

To this end, the present inventor has conducted intensive studies and, as a result, found that symptoms of low-turnover bone diseases can be expressed in an animal, by parathyroidectomy (or destroying the functions thereof) to reduce the blood level of a parathyroid hormone to a physiological level or less, and causing chronic kidney disease while avoiding secondary hyperparathyroidism. Further, on the basis of this finding, the present inventor prepared animal models of low-turnover bone diseases, i.e., animal models characterized in that the parathyroid gland is removed (or the functions thereof destroyed), an amount of a parathyroid hormone is maintained at a physiological level or less by administration thereof, and the kidneys are partially removed (or partial renal artery ligation is carried out). The present inventor used the animal models to carry out a screening of substances having an anti-low-bone-turnover activity, and found that a spherical activated carbon has such a pharmacological activity, to attain the above object. The present invention is based on the above findings.

The present invention relates to an agent for treating or preventing a low-turnover bone disease, comprising as an active ingredient a spherical activated carbon.

The present invention relates to a pharmaceutical composition for treating or preventing a low-turnover bone disease, comprising a spherical activated carbon and a pharmaceutically or veterinarily acceptable carrier or diluent. Hereinafter, the agent of the present invention for treating or preventing a low-turnover bone disease, and the pharmaceutical composition of the present invention for treating or preventing a low-turnover bone disease are collectively referred to as the medicament of the present invention.

According to a preferred embodiment of the medicament of the present invention, the low-turnover bone disease is renal osteodystrophy (particularly renal osteodystrophy caused by a low-turnover bone) or adynamic bone disease (more preferably adynamic bone disease in conservative chronic kidney disease, or renal osteodystrophy in a patient under treatment with dialysis).

According to another preferred embodiment of the medicament of the present invention, a particle size of the spherical activated carbon is 0.01 to 2 mm.

The present invention relates to a method for treating or preventing a low-turnover bone disease, comprising administering to a subject in need thereof a spherical activated carbon in an amount effective thereof.

According to a preferred embodiment of the method of the present invention, the low-turnover bone disease is renal osteodystrophy (particularly renal osteodystrophy caused by a low-turnover bone) or adynamic bone disease (more preferably adynamic bone disease in conservative chronic kidney disease, or renal osteodystrophy in a patient under treatment with dialysis).

According to another preferred embodiment of the method of the present invention, a particle size of the spherical activated carbon is 0.01 to 2 mm.

The present invention relates to the use of a spherical activated carbon in the manufacture of an agent for treating or preventing a low-turnover bone disease or a pharmaceutical composition for treating or preventing a low-turnover bone disease.

According to a preferred embodiment of the use of the present invention, the low-turnover bone disease is renal osteodystrophy (particularly renal osteodystrophy caused by a low-turnover bone) or adynamic bone disease (more preferably adynamic bone disease in conservative chronic kidney disease, or renal osteodystrophy in a patient under treatment with dialysis).

According to another preferred embodiment of the use of the present invention, a particle size of the spherical activated carbon is 0.01 to 2 mm.

BRIEF DESCRIPTION OF THE DRAWINGS

Figure 1 is a graph showing a bone formation rate in rat models.
Figure 2 is a graph showing an effect of a spherical activated carbon in rat models, as a bone formation rate.

BEST MODE FOR CARRYING OUT THE INVENTION

Hereinafter, the medicament, of the present invention, the method of treatment or prevention of the present invention, and the use of the present invention will be explained with reference to the medicament of the present invention, but the following descriptions may be applied to the medicament of the present invention, the method of treatment or prevention of the present invention, and the use of the present invention.

The spherical activated carbon which may be used as the active ingredient of the medicament according to the present invention is not particularly limited, so long as it is a medical spherical activated carbon. As the spherical activated carbon, a spherical activated carbon for an oral administration, i.e., a spherical activated carbon which can be used for medical and internal use is preferable. The particle size of the spherical activated carbon is preferably 0.01 to 2 mm, more preferably 0.05 to 2 mm, most preferably 0.05 to 1 mm.

As the spherical activated carbon, for example, spherical activated carbons disclosed in Japanese Unexamined Patent Publication (Kokai) No. 11-292770 or Japanese Unexamined Patent Publication (Kokai) No. 2002-308785 may be used. Hereinafter, the spherical activated carbon disclosed in JP 11-292770 will be first explained, and then the spherical activated carbon disclosed in JP 2002-308785 explained.

The spherical activated carbon disclosed in JP 11-292770 is a spherical activated carbon having a diameter of preferably 0.05 to 2 mm, more preferably 0.1 to 1 mm. A specific surface area thereof is preferably 500 to 2000 $m^2$/g, more preferably 700 to 1500 $m^2$/g. Further, a volume of voids having a pore radius of 100 to 75000 angstrom is preferably 0.01 to 1 mL/g, more preferably 0.05 to 0.8 mL/g. In this connection, the specific surface area is measured by a methanol adsorption method using an automatic adsorption measuring apparatus. The void volume is measured by a mercury press-injection porosimeter. The spherical activated carbon has advantages in that it can be used as a dose without scattering, and that constipation is not caused by a multiple administration, in comparison with a powdery activated carbon.

The shape of the spherical activated carbon is an important factor, and a substantially spherical shape is important. Among known spherical activated carbons, a spherical activated carbon obtained from a petroleum pitch, as described below, is most preferable, because it is close to a completely spherical shape.

In the manufacture of the spherical activated carbon disclosed in JP 11-292770, any active carbon materials, such as sawdust, coal, coconut shell, petroleum or coal pitches, or organic synthetic polymers, may be used. The spherical activated carbon may be produced, for example, by carbonizing the material and activating the carbonized substance. The activation may be performed by, for example, a steam-activation method, a chemical activation method, an air-activation method, or a $CO_2$ activation method, so long as a medically acceptable purity is maintained.

As the spherical activated carbon disclosed in JP 11-292770, there may be mentioned, for example, a granulated active carbon obtained from carbonaceous powder, a spherical activated carbon prepared from an organic polymer, or a spherical activated carbon obtained from a petroleum hydrocarbon (a petroleum pitch).

The granulated active carbon obtained from carbonaceous powder can be prepared, for example, by the following procedure. A binder such as tar or pitch is used to granulate a carbonaceous powdery material. The resulting microspherical shaped substance is carbonized (or calcinated) by heat-treatment at 600 to 1000°C in an inert atmosphere to be carbonized. Further, the carbonized substance is activated to obtain the granulated active carbon. The activation may be performed by, for example, a steam-activation method, a chemical activation method, an air-activation method, or a $CO_2$ activation method. The steam-activation can be performed at 800 to 1100°C in an atmosphere of steam.

The spherical activated carbon prepared from an organic polymer is disclosed in, for example, Japanese Examined Patent Publication (Kokoku) No. 61-1366, and may be prepared by the following procedure. A condensation-type or polyaddition-type thermosetting prepolymer is mixed with a curing agent, a curing catalyst, and an emulsifying agent. The mixture is emulsified in water with stirring, and reacted at room temperature or at a higher temperature with stirring. The reaction system becomes a suspension, and then a spherical thermosetting polymer is generated with stirring. The product is collected, and heated at 500°C or more in an inert atmosphere to be carbonized. The carbonized substance is activated by the above-mentioned method to obtain the spherical activated carbon.

The spherical activated carbon obtained from a petroleum pitch has a diameter of preferably 0.05 to 2 mm, more preferably 0.1 to 1 mm, a specific surface area of preferably 500 to 2000 $m^2$/g, more preferably 700 to 1500 $m^2$/g, and a volume of voids having a pore radius of 100 to 75000 angstrom of preferably 0.01 to 1 mL/g. The spherical activated carbon obtained from a petroleum pitch may be prepared, for example, by the following two methods.

The first method is disclosed in, for example, Japanese Examined Patent Publication (Kokoku) No. 51-76 (United States Patent No. 3917806) or Japanese Unexamined Patent Publication (Kokai) No. 54-89010 (United States Patent No. 4761284). In the first method, a pitch is granulated under a melted condition, and treated with oxygen. The resulting infusible substance is heated at 600 to 1000°C in an inert atmosphere to be carbonized. Further, the carbonized substance is activated at 850 to 1000°C in an atmosphere of steam. The second method is disclosed in, for example, Japanese Examined Patent Publication (Kokoku) No. 59-10930 (United States Patent No. 4420433). In the second method, a pitch is formed into string-like shaped products under a melted condition. The string-like shaped products are broken and added to hot water to be spheroidized. An oxygen treatment is performed to obtain an infusible substance, and the carbonization and activation are carried out by the same methods as described in the first method.

As the spherical activated carbon which may be used as the active ingredient in the present invention, (1) a spherical activated carbon treated with ammonia, or (2) a spherical activated carbon treated with an oxidation treatment and/or a reduction treatment, may be used. The treatments can be applied to, for example, the spherical activated carbon obtained from a petroleum pitch, the granulated active carbon obtained from carbonaceous powder, or the spherical activated carbon prepared from an organic polymer, as described above.

In the ammonia treatment, for example, a spherical activated carbon is treated in an aqueous ammonia solution (1 to 1000 ppm; a volume ratio of the aqueous ammonia solution to the spherical activated carbon = 2 to 10) at 10 to 50°C for 0.5 to 5 hours. A spherical activated carbon obtained by treating a spherical activated carbon derived from a petroleum pitch with ammonia is disclosed in, for example, Japanese Unexamined Patent Publication (Kokai) No. 56-5313 (United States Patent No. 4761284). As the spherical activated carbon treated with ammonia, there may be mentioned, for example, a spherical activated carbon having a diameter of 0.05 to 2 mm, preferably 0.1 to 1 mm, a specific surface area of 500 to 2000 $m^2$/g, preferably 700 to 1500 $m^2$/g, a volume of voids having a pore radius of 100 to 75000 angstrom of 0.01 to 1 mL/g, and a pH of 6 to 8.

The above-mentioned oxidation treatment means a heat treatment at a high temperature in an oxidative atmosphere containing oxygen. As an oxygen source, for example, pure oxygen, nitrogen oxide, or air can be used. The above-mentioned reduction treatment means a heat treatment at a high temperature in an inert atmosphere with respect to carbon. The inert atmosphere with respect to carbon can be formed by using nitrogen gas, argon gas, or helium gas, or a mixed gas thereof.

The oxidation treatment is carried out in an atmosphere containing preferably 0.5 to 25% by volume, more preferably 3 to 10% by volume of oxygen at preferably 300 to 700°C, more preferably 400 to 600°C. The reduction treatment is carried out in an inert atmosphere at preferably 700 to 1100°C, more preferably 800 to 1000°C.

A spherical activated carbon obtained by treating a spherical activated carbon derived from a petroleum pitch with the oxidative treatment and/or the reduction treatment is disclosed in, for example, Japanese Examined Patent Publication (Kokoku) No. 62-11611 (United States Patent No. 4681764).

As the spherical activated carbon treated with the oxidative treatment and/or the reduction treatment, a spherical activated carbon having a diameter of 0.05 to 2 mm, preferably 0.1 to 1 mm, a specific surface area of 500 to 2000 $m^2$/g, preferably 700 to 1500 $m^2$/g, and a volume of voids having a pore radius of 100 to 75000 angstrom of 0.01 to 1 mL/g is preferable.

The spherical activated carbon disclosed in JP 2002-308785 is a spherical activated carbon having a diameter of 0.01 to 1 mm, a specific surface area determined by a BET method of 700 $m^2$/g or more, a volume of pores having a pore diameter of 20 to 15000 nm ranges from not less than 0.04 mL/g to less than 0.10 mL/g, a total amount of acidic groups of 0.30 to 1.20 meq/g, and a total amount of basic groups of 0.20 to 0.70 meq/g. The spherical activated carbon disclosed in JP 2002-308785 has a specific range of pore volume, namely, a volume of pores having a pore diameter of 20 to 15000 nm is from not less than 0.04 mL/g to less than 0.10 mL/g. Further, a spherical activated carbon having a total amount of basic groups of 0.20 to 1.00 meq/g (see Japanese Patent Application No. 2002-293906 or Japanese Patent Application No. 2002-293907) may be used in the present invention.

In the spherical activated carbon disclosed in JP 11-292770, a volume of voids having a pore radius of 100 to 75000 angstrom, i.e., a volume of pores having a pore diameter of 20 to 15000 nm, is 0.1 to 1 mL/g. According to the disclosures in JP 2002-308785, when the volume of pores having a pore diameter of 20 to 15000 nm is adjusted to a range of from not less than 0.04 mL/g to less than 0.10 mL/g, an adsorbability of $\alpha$-amylase that is a useful substance, is significantly lowered, while maintaining a high adsorbability of $\beta$-aminoisobutyric acid, that is a toxic substance. When the volume of pores having a pore diameter of 20 to 15000 nm is increased, the useful substances such as digestive enzymes are more easily adsorbed. Therefore, a smaller volume of pores having a pore diameter of 20 to 15000 nm is preferable from a viewpoint that an adsorption of useful substances is reduced. On the other hand, if the volume of pores having such a pore diameter becomes too small, the adsorption of harmful substances is lowered. Therefore, in the adsorbent for an oral administration, a ratio (T/U) of an adsorption amount (T) of toxic substances to an adsorption amount (U) of useful substances, that is, a selective adsorption rate, is important. For example, the selective adsorption rate of the spherical activated carbon can be evaluated by the ratio (Tb/Ua) of an adsorption amount (Tb) of DL-$\beta$-aminoisobutyric acid (toxic substance) to an adsorption amount (Ua) of $\alpha$-amylase (useful substance). More particularly, the selective adsorption rate can be evaluated by, for example, an equation:

$$A = Tb/Ua$$

wherein A denotes a selective adsorption rate, Tb denotes an adsorption amount of DL-$\beta$-aminoisobutyric acid, and Ua denotes an adsorption amount of $\alpha$-amylase.

The spherical activated carbon disclosed in JP 2002-308785 exhibits an excellent selective adsorption rate when the volume of pores having a pore diameter of 20 to 15000 nm ranges from not less than 0.04 mL/g to less than 0.10 mL/g, and a more excellent selective adsorption rate when the volume of pores having a pore diameter of 20 to 15000 nm ranges from not less than 0.05 mL/g to less than 0.10 mL/g.

The spherical activated carbon disclosed in JP 2002-308785 has a diameter of 0.01 to 1 mm, preferably 0.02 to 0.8 mm. In this connection, the expression that "a diameter is Dl to Du" as used herein means that a screen passing percentage (%) in a range of a screen opening Dl to Du is 90% or more in a particle-sizes accumulating standard curve prepared in accordance with JIS K 1474 as mentioned below in relation to a method for determining an average particle diameter.

The spherical activated carbon disclosed in JP 2002-308785 has a specific surface area (referred to as "SSA" hereinafter) determined by a BET method of 700 m$^2$/g or more. When the spherical activated carbon has an SSA of less than 700 m$^2$/g, an adsorbability of toxic substances is lowered. The SSA is preferably 800 m$^2$/g or more. The upper limit of the SSA is not particularly limited, but the SSA is preferably 2500 m$^2$/g or less in view of a bulk density and strength.

The spherical activated carbon disclosed in JP 2002-308785 has a special constitution of functional groups, that is, a total amount of acidic groups is 0.30 to 1.20 meq/g, and a total amount of basic groups is 0.20 to 0.70 meq/g. When the spherical activated carbon does not satisfy the functional-groups requirement, i.e., the total amount of acidic groups is 0.30 to 1.20 meq/g and the total amount of basic groups is 0.20 to 0.70 meq/g, the adsorbability of the harmful substances is lowered. In the functional-groups requirement, the total amount of acidic groups is preferably 0.30 to 1.00 meq/g and the total amount of basic groups is preferably 0.30 to 0.60 meq/g. A preferable functional-groups constitution is that the total amount of acidic groups is 0.30 to 1.20 meq/g, the total amount of basic groups is 0.20 to 0.70 meq/g, a phenolic hydroxyl group is 0.20 to 0.70 meq/g, and a carboxyl group is 0.15 meq/g or less, and a ratio (a/b) of the total amount of acidic groups (a) to the total amount of basic groups (b) is 0.40 to 2.5, and a relation [(b+c)-d] between the total amount of basic groups (b), the phenolic hydroxyl group (c), and the carboxyl group (d) is 0.60 or more.

The spherical activated carbon disclosed in JP 2002-308785 may be prepared by, for example, the following methods.

First, a dicyclic or tricyclic aromatic compound or a mixture thereof having a boiling point of 200°C or more is added as an additive to a pitch such as a petroleum pitch or a coal pitch. The whole is heated and mixed, and then shaped to obtain a shaped pitch. The spherical activated carbon is for oral administration, and the raw material must have a sufficient purity from a safety standpoint, and have stable properties.

Thereafter, the shaped pitch is dispersed and granulated in hot water at 70 to 180°C, with stirring, to obtain a microspherical shaped pitch. Further, the additive is extracted and removed from the shaped pitch by a solvent having a low solubility to the pitch but a high solubility to the additive. The resulting porous pitch is oxidized by an oxidizing agent to obtain a porous pitch having an infusibility to a heat. The resulting infusible porous pitch is treated at 800 to 1000°C in a gas flow such as steam or carbon dioxide gas reactive with carbon to obtain a porous carbonaceous substance.

Then, the resulting porous carbonaceous substance is oxidized in a temperature range of 300 to 800°C, preferably 320 to 600°C in an atmosphere containing 0.1 to 50% by volume, preferably 1 to 30% by volume, particularly preferably 3 to 20% by volume of oxygen, and thereafter reduced in a temperature range of 800 to 1200°C, preferably 800 to 1000°C, in an atmosphere of a non-oxidizable gas to obtain the spherical activated carbon disclosed in JP 2002-308785.

In the above method, the atmosphere containing oxygen in the particular amount may be pure oxygen, or nitrogen oxides or air as the oxygen source. As the atmosphere inert against carbon, for example, nitrogen, argon or helium may be used alone or in the form of a mixture thereof.

The purposes of the addition of the aromatic compound to the raw pitch are that a flowability of the raw pitch is enhanced by lowering a softening point of the raw pitch whereby the granulation thereof is made easier, and the porous pitch is produced by extracting and removing the additive from the shaped pitch, whereby a structure control and a calcination of the carbonaceous material by oxidization in the subsequent steps is made easier. As the additive, for example, naphthalene, methylnaphthalene, phenyl-naphthalene, benzyl-naphthalene, methylanthracene, phenanthrene, or biphenyl may be used alone or in a mixture thereof. An amount of the additive added to the pitch is preferably 10 to 50 parts by weight of the aromatic compound with respect to 100 parts by weight of the pitch.

It is preferable that the pitch and the additive are mixed under a melted condition with heating, to achieve a homogeneous mixing. Further, it is preferable that the mixture of the pitch and the additive is shaped to form particles having a particle size of about 0.01 to 1 mm, to control the particle size (diameter) of the resulting porous spherical carbonaceous substance. The shaping may be conducted during the melted condition, or by grinding the mixture after it has cooled.

A preferable solvent used to extract and remove the additive from the mixture of the pitch and the additive may be, for example, an aliphatic hydrocarbon, such as butane, pentane, hexane, or heptane, a mixture comprising an aliphatic hydrocarbon as a main component, such as naphtha or kerosene, or an aliphatic alcohol, such as methanol, ethanol, propanol, or butanol.

The additive may be removed from the shaped mixture by extracting the additive with the solvent from the shaped mixture of the pitch and the additive, while maintaining the shape. It is assumed that, upon the extraction, through-holes of the additive are formed in the shaped product, and a shaped pitch having a uniform porosity can be obtained.

In this connection, the size of through-holes of the additive (i.e., pore volume) may be controlled by a conventional method, for example, by controlling an amount of the additive, or a precipitating temperature (cooling temperature) of the additive in the granulating step of the shaped pitch. Further, when the resulting shaped pitch is crosslinked by oxidation, the pore volume generated by extracting the additive is affected by a condition of the treatment. For example, if it is strongly crosslinked by oxidation, a heat contraction caused by a heat treatment is small, and thus the pores obtained by extracting the additive tend to be maintained.

Then, the resulting porous shaped pitch is crosslinked by oxidation, that is, the resulting porous shaped pitch is oxidized by an oxidizing agent, preferably at room temperature to 300°C to obtain the porous infusible shaped pitch having a non-fusibility to heat. As the oxidizing agent, for example, oxygen gas ($O_2$), or a gas mixture prepared by diluting oxygen gas ($O_2$) with air or nitrogen may be used.

Properties of the spherical activated carbon disclosed in JP 2002-308785, namely, the average particle diameter, the specific surface area, the pore volume, the total amount of acidic groups, and the total amount of basic groups are measured by the following methods.

(1) Average particle diameter

A particle-sizes accumulating standard curve is prepared in accordance with JIS K 1474 for the spherical activated carbon. The average particle diameter is determined from a screen opening (mm) at an intersection point with a line that is horizontal to an abscissa axis and starts from an intersection point in the particle-sizes accumulating standard curve with a perpendicular line from a 50% point of the abscissa axis.

(2) Specific surface area

An amount of gas adsorbed is measured by a specific surface area measuring apparatus (for example, Flow Sorb II 2300 manufactured by MICROMERITICS) in accordance with a gas adsorbing method of a continuous flow for the spherical activated carbon sample, and a specific surface area can be calculated by a BET equation. More particularly, the spherical activated carbon is charged as a sample in a sample tube. A helium gas stream containing 30% by volume of nitrogen is passed through the sample tube, and an amount of nitrogen adsorbed to the spherical activated carbon sample is measured by the following procedures. Specifically, the sample tube is cooled to -196°C, whereby nitrogen is adsorbed to the spherical activated carbon sample, and then the temperature of the sample tube is raised to room temperature. During the raising of the temperature, nitrogen is emitted from the spherical activated carbon sample. The amount of emitted nitrogen is measured by a heat conductivity type detector as an amount (v) of gas adsorbed.

A value $v_m$ is calculated in accordance with a one-point method (relative pressure x = 0.3) by a nitrogen adsorption at a temperature of liquid nitrogen, using an approximate equation:

$$v_m = 1/(v \cdot (1-x))$$

derived from the BET equation. Then, a specific surface area of the sample is calculated by an equation:

$$\text{specific surface area} = 4.35 \times v_m (m^2/g).$$

In the above equations, $v_m$ is an adsorption amount ($cm^3/g$) necessary to form a monomolecular layer on a surface of the sample, v is an adsorption amount ($cm^3/g$) actually found, and x is a relative pressure.

(3) Pore volume by a mercury injection method

The pore volume can be measured by a mercury press-injection porosimeter (for example, AUTOPORE 9200 manufactured by MICROMERITICS). The spherical activated carbon is charged as a sample in a sample vessel, and degassed under a pressure of 2.67Pa or less for 30 minutes. Then, mercury is introduced into the sample vessel, and a pressure applied is gradually increased (maximum pressure = 414 MPa) to force the mercury into the micropores in the spherical activated carbon sample. A pore volume distribution of the spherical activated carbon sample is measured from a relationship between the pressure and an amount of forced mercury by equations as mentioned below. Specifically, a volume of mercury inserted into the spherical activated carbon sample while a pressure is applied is increased from a pressure (0.07 MPa) corresponding to a pore diameter of 15 μm to the maximum pressure (414 Mpa) corresponding to a pore diameter of 3 nm. A pore diameter can be calculated as follows. When mercury is forced into a cylindrical micropore having a diameter (D) by applying a pressure (P), a surface tension (γ) of mercury is balanced with a pressure acting on a section of the micropore, and thus, the following equation is held:

$$-\pi D\gamma\cos\theta = \pi(D/2)^2 \cdot P$$

wherein θ is a contact angle of mercury and a wall of the micropore. Therefore, the following equation:

$$D = (-4\gamma\cos\theta)/P$$

is held.

In the present specification, the relationship between the pressure (P) and the pore diameter (D) is calculated by an equation:

$$D = 1.27/P$$

given that a surface tension of mercury is 484 dyne/cm, a contact angle of mercury and carbon is 130°, a unit of the pressure P is Mpa, and a unit of the pore diameter D is μm. The volume of pores having a pore diameter of 20 to 15000 nm in the present invention corresponds to a volume of mercury inserted by applying a pressure increasing from 0.07 Mpa to 63.5 Mpa.

(4) Total amount of acidic groups

The total amount of acidic groups is an amount of NaOH consumed, which may be determined by adding 1 g of the spherical activated carbon sample, after being crushed to form particles having a size of less than 200 mesh, to 50 mL of a 0.05N NaOH solution; shaking the mixture for 48 hours; then filtering out the spherical activated carbon sample; and titrating until neutralization.

(5) Total amount of basic groups

The total amount of basic groups is an amount of HCl consumed, which may be determined by adding 1 g of the spherical activated carbon sample after being crushed to form particles having a less than 200 mesh size, to 50 mL of a 0.05N HCl solution; shaking the mixture for 24 hours; then filtering out the spherical activated carbon sample; and titrating until neutralization.

The medicament of the present invention is useful in treating or preventing low-turnover bone diseases, i.e., various diseases caused by a low-turnover bone, such as renal osteodystrophy (particularly renal osteodystrophy caused by a low-turnover bone) or adynamic bone disease. The adynamic bone disease includes adynamic bone disease in conservative chronic kidney disease, and renal osteodystrophy in a patient under treatment with dialysis.

A metabolism in bone is performed as the whole of bone tissues, in the balance of osteogenesis and bone resorption. The term "low-turnover bone" as used herein means conditions in which osteogenesis is lowered in comparison with the normal level, and thus, bone resorption is also lowered in comparison with the normal level.

The term "adynamic bone disease" as used herein means conditions in which chronic kidney disease progresses, and as a result, the metabolism in bone (bone turnover) is inhibited, and osteogenesis is extremely inhibited, dependent on the progress of chronic kidney disease. The "adynamic bone disease" includes conditions in which no osteogenesis occurs, and conditions in which osteogenesis is extremely inhibited.

The spherical activated carbon (preferably having a particle size of 0.01 to 2 mm) which may be used as the active ingredient of the medicament of the present invention may be administered alone or, optionally together with a pharmaceutically or veterinarily acceptable ordinary carrier or diluent, to a subject (an animal, preferably a mammal, particularly a human) in need of a treatment or prevention of various diseases caused by a low-turnover bone, in an amount effective thereof. Preferably, the medicament of the present invention may be orally administered. The dose depends on, for example, the kind of subject (a mammal, particularly a human), the age, individual differences, and/ or symptoms of the subject. For example, when the subject is a human, the dose is normally 0.2 to 20 g of spherical activated carbon per day. The dose may be appropriately changed in accordance with the symptoms. Further, the dose may be administered as a single dose or a multiple dose. The spherical activated carbon per se may be administered, or it may be administered as a pharmaceutical composition containing the spherical activated carbon. In the former, the spherical activated carbon may be administered as a slurry prepared by suspending it in drinking water.

The formulation may be administered in any form, such as granules, tablets, sugar-coated tablets, capsules, sticks, divided packages, or suspensions. In the case of capsules, the usual gelatin capsules, or if necessary, enteric capsules may be used. In the case of granules, tablets, or sugar-coated tablets, the formulations must be broken into the original fine particles inside the body. The content of the spherical activated carbon in the medicament is normally 1 to 100%. The preferred medicaments are capsules, sticks, or divided packages, and the spherical activated carbon per se may be packed into a package.

The pharmacological activities in the medicament of the present invention may be confirmed by, for example, animal models prepared in Examples. Such an animal model, which may be used in evaluating the medicament of the present invention, may be prepared by, for example, the following procedure.

The animal model is not particularly limited, so long as it may be used in analyzing a mechanism or factors in low-turnover bone diseases (such as adynamic bone disease, or renal osteodystrophy caused by a low-turnover bone) and confirming pharmacological activities to low-turnover bone. As the animal model, there may be mentioned, for example, a rat, a mouse, a guinea pig, a hamster, a rabbit, a dog, or a miniature pig, and a rat is most preferable. When a rat is used, a 3 to 20-week-old, particularly 8 to 12-week-old rat is preferable.

The thyroidectomy and parathyroidectomy in the preparation of the animal model can be performed by, for example, cutting the center line in an area including the submandibular gland in the neck of the animal, and removing the thyroid gland on the trachea. In this connection, the parathyroid gland exists on the thyroid gland, and is integrated into the thyroid gland. Instead of the removal, the thyroid and parathyroid glands may be burned by an electric knife or a laser, or the functions thereof may be lost by a denaturation of protein using alcohol or formalin. If possible, it is preferable to maintain the thyroid gland and to remove (or cause a loss of functions) only the parathyroid gland. Hereinafter, an animal in which the thyroid and parathyroid glands are removed is represented as "TPTx". For example, a rat in which the thyroid and parathyroid glands are removed is represented as a TPTx rat.

Normal physiological amounts of thyroid hormones (such as T3 or T4) in blood of the animal model (rat) are 0.1 to 1 ng/mL (T3) and 1 to 100 ng/dL (T4) [preferably 0.3 to 0.8 ng/mL (T3) and 10 to 30 ng/dL (T4)], and that of a parathyroid hormone is 0 to 1000 pg/mL, preferably 0 to 150 pg/mL (by an ELISA method).

Thyroid and parathyroid hormones may be administered in the form of an aqueous solution such as a buffer or a physiological saline. After preparing the animal model, the concentrations of the thyroid and parathyroid hormones in the blood of the animal model are maintained so that T3 is 0.1 to 1 ng/mL and T4 is 1 to 100 ng/dL (preferably T3 is 0.3 to 0.8 ng/mL and T4 is 10 to 30 ng/dL), and the parathyroid hormone is a physiological amount or less (0 to 1000 pg/mL), preferably 0 to 150 pg/mL (by an ELISA method).

The partial removal of kidneys may be performed, for example, by the following procedure. A rat is retained in a supine position, and an anesthetic such as sodium pentobarbital (Nembutal; Dainippon Pharmaceutical Co., Ltd.) is administered to the right lower quadrant of the abdomen (50 mg/Kg.BW). Under anesthesia, hair on both the left and right sides of the abdomen is shaved. To remove the right kidney, the rat is retained so that the right side thereof faces to the operator. After an area to be incised is sterilized, the abdominal wall approximately 10 mm apart from the edges of ribs is incised approximately 15 mm along the ribs. The right kidney is separated from fat therearound and the adrenal gland. After renal blood vessels and the ureter are ligated towards the kidney side and artery side to avoid a change in weight of the kidney caused by bleeding, the right kidney is removed. The wet weight of the right kidney is measured to determine a standard weight when the left kidney is removed. The incised abdominal wall and skin are sutured.

The rat is retained so that the left side thereof faces to the operator. After an area to be incised is sterilized, the abdominal wall approximately 10 mm apart from the edges of ribs is incised approximately 15 mm along the ribs. The left kidney is separated from fat therearound and the adrenal gland. For hemostasis, the renal arteries and renal veins are clamped using a 5-0 suture. The left kidney is placed in a mold for severing kidney, and severed. Assuming that the weight of the right kidney is equal to that of the left kidney, a proportion of the severance is calculated on the basis of the weight of the removed right kidney. After the kidney is severed, 2500 Unit/mL of thrombin (SIGMA) is added to the severed surface for hemostasis.

After the left kidney is replaced in the abdomen, one or two drops of penicillin (GIBCO) are added, and the abdominal wall and skin are sutured. Dilute iodine tincture (Yoshida Pharmaceutical) is applied to the right and left sutured surfaces for sterilization. After 2 days from the preparation of the animal models, Cr (creatinine in serum) and BUN (urea nitrogen in serum) are measured to exclude inadequate animal models. After 2 to 6 weeks from the preparation, animal models are evenly divided into groups, on the basis of the weight, Cr, BUN, an amount of Cr to be excreted, Ccr (creatinine clearance), an amount of proteins to be excreted, blood pressure, and the proportion of severance.

Next, the renal artery ligation method will be explained. The procedure of the left renal partial artery ligation is, for example, as follows. A rat is retained in a supine position, and sodium pentobarbital (Nembutal; Dainippon Pharmaceutical Co., Ltd.) is administered to the right lower quadrant of the abdomen (50 mg/Kg.BW). Under anesthesia, hair on the side of the left kidney is shaved by hair clippers. The rat is retained so that the left side thereof faces to the operator. After an area to be incised is sterilized, the abdominal wall approximately 10 mm apart from the edges of ribs is incised approximately 15 mm along the ribs. The abdominal muscle layer at the right side of the incised area is lifted with forceps with rings, and the left kidney is produced from the body while pressing the kidney gently with the left hand. Fat around the kidney is removed with small scissors. Microsurgery forceps are used to stop the blood flow, and renal arterial branches to be maintained are determined. Microsurgery forceps are inserted into the boundary between the renal arteries and renal veins to be separated. Renal arteries other than those to be maintained are ligated with a 7-0 suture. Fat tissues around the separated kidney are removed, and the kidney is replaced in the abdomen. After one or two drops of penicillin (GIBCO) is added, and the abdominal wall and skin are sutured. Dilute iodine tincture (Yoshida Pharmaceutical) is applied to the left sutured surface for sterilization.

The procedure of total ligation of right renal artery is, for example, as follows. Renal artery ligation is carried out after 1 week from the left branch artery ligation. A rat is retained in a supine position, and sodium pentobarbital (Nembutal; Dainippon Pharmaceutical Co., Ltd.) is administered to the right lower quadrant of the abdomen (50 mg/Kg.BW). Under anesthesia, hair on the side of the right kidney is shaved by hair clippers. The rat is retained so that the right side thereof faces to the operator. After an area to be incised is sterilized, the abdominal wall approximately 10 mm apart from the edges of ribs is incised approximately 15 mm along the ribs. The abdominal muscle layer at the right side of the incised area is lifted with forceps with rings, and the right kidney is produced from the body while pressing the kidney gently with the left hand. The right forefinger and thumb are used to break a portion of capsule, and the kidney is produced from the capsule. The hilum renalis is clamped with forceps, and the portion therebelow is ligated with a 1-0 suture. After the forceps are released, the right kidney is replaced in the abdomen, one or two drops of penicillin (GIBCO) are added, and the abdominal wall and skin are sutured. Dilute iodine tincture (Yoshida Pharmaceutical) is applied to the right sutured surface for sterilization.

After 2 days from the preparation of the animal models, the weight, Cr, and BUN are measured to exclude inadequate animal models. After 2 to 6 weeks from the preparation, animal models are evenly divided into groups, on the basis of the weight, Cr, BUN, an amount of Cr to be excreted, Ccr, an amount of proteins to be excreted, and blood pressure.

In this connection, the proportion of kidney to be removed may be appropriately determined. When a rat is used, it is preferable to remove, for example, two-thirds of the left kidney and the whole of the right kidney (hereinafter referred to as 5/6Nx), a half of the left kidney and the whole of the right kidney (3/4Nx), or the whole of the right kidney (1/2Nx).

The pharmacological activities in the medicament of the present invention may be evaluated by, for example, the following procedure.

The obtained animal models are bred until the bone formation rate is significantly lowered. When a rat is used as the animal model, it is preferable to start the evaluation after 2 to 8 weeks (particularly 4 to 6 weeks) from the partial removal of kidneys or partial renal artery ligation. In this connection, as a control group, an animal in which the kidneys are maintained but only the parathyroidectomy is carried out, an animal in which the kidneys are maintained but the thyroidectomy and parathyroidectomy are carried out (TPTx), or an animal in which the kidneys are maintained and the thyroidectomy and parathyroidectomy are not carried out, may be used. A TPTx in which only the incision treatment in the above procedure of kidney removal is carried out (TPTx-sham) is preferable.

In the above evaluation procedure, a high-calcium diet is fed to the control group and the animal models. The diet generally contains 0.4 to 2% (preferably 1 to 2%) of calcium.

EXAMPLES

The present invention now will be further illustrated by, but is by no means limited to, the following Examples.

Preparative Example 1: Preparation of spherical activated carbon

Petroleum pitch (68 kg) (softening point = 210°C; quinoline insoluble contents = 1% or less by weight; ratio of hydrogen atoms/carbon atoms = 0.63) and naphthalene (32 kg) were charged into an autoclave (internal volume = 300 L) equipped with stirring fans, melted at 180°C, and mixed. The mixture was extruded at 80 to 90°C to form string-like shaped products. Then, the string-like shaped products were broken so that a ratio of a diameter to a length became about 1 to 2.

The resulting broken products were added to an aqueous solution containing 0.23% by weight of polyvinyl alcohol (saponification value = 88%) and heated to 93°C, and dispersed with stirring to be spheroidized. Then, the whole was cooled by replacing the polyvinyl alcohol aqueous solution with water, at 20°C for 3 hours, whereby the pitch was solidified and naphthalene crystals were precipitated, and a slurry of spherical shaped products of pitch was obtained.

After most of the water was removed by filtration, naphthalene in pitch was extracted and removed with n-hexane at an amount about 6 times that of the spherical shaped products of pitch. The resulting porous spherical pitch was heated to 235°C by passing a heated air in a fluidized bed, and allowed to stand at 235°C for 1 hour to be oxidized, and a porous spherical oxidized pitch was obtained, which is nonfusible to heat.

Thereafter, the resulting porous spherical oxidized pitch was activated in a fluidized bed at 900°C for 170 minutes by a nitrogen gas atmosphere containing 50% by volume of steam to obtain a porous spherical activated carbon. Further, the resulting spherical activated carbon was oxidized in a fluidized bed at 470°C for 3 hours and 15 minutes by a nitrogen-oxygen atmosphere containing 18.5% by volume of oxygen, and reduced in a fluidized bed at 900°C for 17 minutes by a nitrogen gas atmosphere, to obtain a spherical activated carbon.

The properties of the resulting spherical activated carbon are as follows:

SSA: 1300 $m^2$/g
Pore volume: 0.08 mL/g
Average particle diameter: 350 $\mu$m
Total amount of acidic groups: 0.67 meq/g
'Total amount of basic groups: 0.54 meq/g

In this connection, the pore volume was determined by a mercury injection method and corresponds to a volume of pores having a diameter of 20 to 15000 nm.

Referential Example 1: Preparation of animal model of adynamic bone disease in chronic kidney disease

Male SD rats (10 weeks old) were used to carry out thyroidectomy and parathyroidectomy (TPTx), and a physiological amount of a thyroid hormone (T4; 1 to 10 $\mu$g/Kg; three times per week; subcutaneous administration) and a physiological amount of a parathyroid hormone (0.1 $\mu$g/kg/hr; continuous subcutaneous administration by an osmotic mini-pump) were administered to the rats so that the concentrations thereof in blood were maintained within normal levels.

In this connection, the parathyroid hormone (PTH) was administered with a constant flow rate per hour by an infusion pump method. In the infusion pump method, a small-sized osmotic mini-pump was subcutaneously implanted, and a liquid was infused from the pump when the pump was soaked with body fluid. Therefore, the PTH dose per day was calculated on the basis of an amount of infused liquid per day, and a concentration of PTH contained in the liquid was determined.

Thereafter, kidneys were removed from the rats to prepare animal models of chronic kidney disease as follows:

5/6Nx: Two-thirds of the left kidney and the whole of the right kidney were removed.
3/4Nx: A half of the left kidney and the whole of the right kidney were removed.
1/2Nx: The whole of the right kidney was removed.

In this referential example, osteogenesis in normal rats, the TPTx rats (only thyroidectomy and parathyroidectomy), the 5/6Nx rats, the 3/4Nx rats, and the 1/2Nx rats were examined. Each group was composed of ten rats. After the rats were bred for 6 weeks from the removal of kidneys, a change in osteogenesis was evaluated in accordance with the following procedure. Calcein, a fluorescent reagent for labeling a calcified portion of bone, was administered to the rats subcutaneously or intraperitoneally at a dose of 8 to 15 mg/kg twice with an interval of one week. After the rats were sacrificed, undecalcified specimens (or sections) of tibia were prepared and bone histomorphometric analysis was performed by image processing and calculate a bone formation rate.

As apparent from serum biochemical tests shown in Table 1, the 5/6Nx rat exhibited the severest pathosis, the 3/4Nx rat exhibited the second severest pathosis, and the 1/2Nx rat exhibited the lightest pathosis,,with respect to renal functions in the rat models after 6 weeks from the removal of kidneys.

Table 1

|  | Normal | TPTx | TPTx-1/2Nx | TPTx-3/4Nx | TPTx-5/6Nx |
|---|---|---|---|---|---|
| Cre(mg/dL) | 0.60±0.00 | 0.69±0.17 | 0.70±0.10 | 1.04±0.15* | 1.32±0.34*# |
| BUN(mg/dL) | 21.8±5.4 | 22.0±5.8 | 26.3±5.0 | 54.8±10.8* | 69.0±25.9*# |

* p<0.05 vs normal rat, # p<0.05 vs TPTx
Cre: creatinine in serum, BUN: urea nitrogen in serum

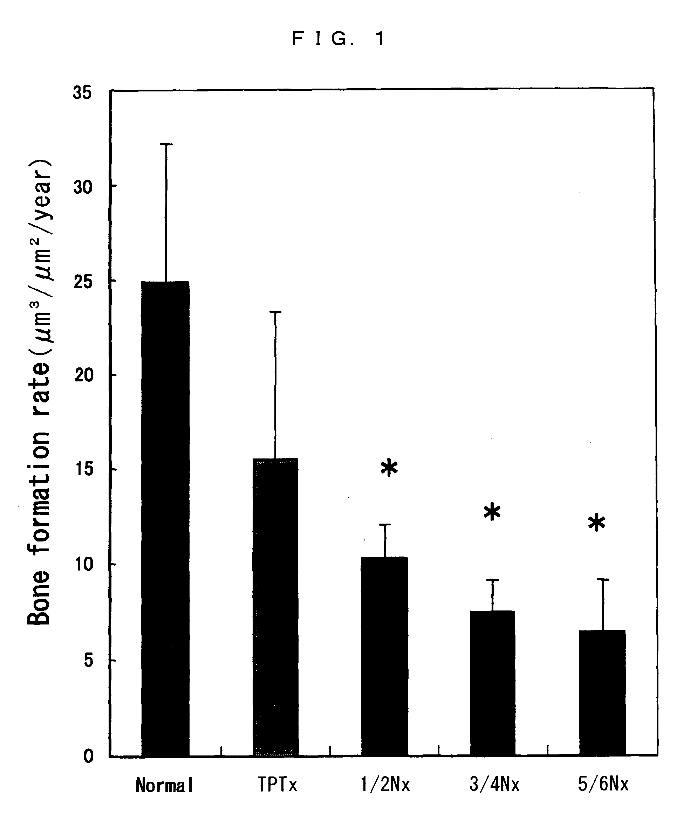

Next, osteogenesis in the tibia of each rat after 6 weeks from the removal of kidneys was compared. The result is shown in Figure 1. The symbol "*" in Figure 1 means p<0.05 with respect to the normal rat.

As shown in Figure 1, it was found that as animal models exhibited a more severe chronic kidney disease, the bone formation rate became significantly lower. As apparent from the result, when chronic kidney disease progresses, the bone turnover is inhibited and lowered, and adynamic bone disease develops. In this reference example, it was found that an animal model of low-turnover bone diseases (particularly adynamic bone disease, or renal osteodystrophy caused by a low-turnover bone) can be prepared.

Example 1: Evaluation for effects of spherical activated carbon using animal models

The animal model of adynamic bone disease described in Reference Example 1 was used to examine therapeutic effects of the parathyroid hormone.

As the animal model, the TPTx-5/6Nx rat prepared in Referential Example 1 was used. After 6 weeks (a bone formation rate was significantly lowered) from the removal of kidneys, a normal feed was changed to a mixed feed prepared by mixing the spherical activated carbon prepared in Preparative Example 1 with the normal feed, and the spherical activated carbon was administered at a dose of 4 g/kg. Each group was composed of ten rats. After the administration for 4 weeks, osteogenesis was evaluated and examined in accordance with the procedure described in Referential Example 1.

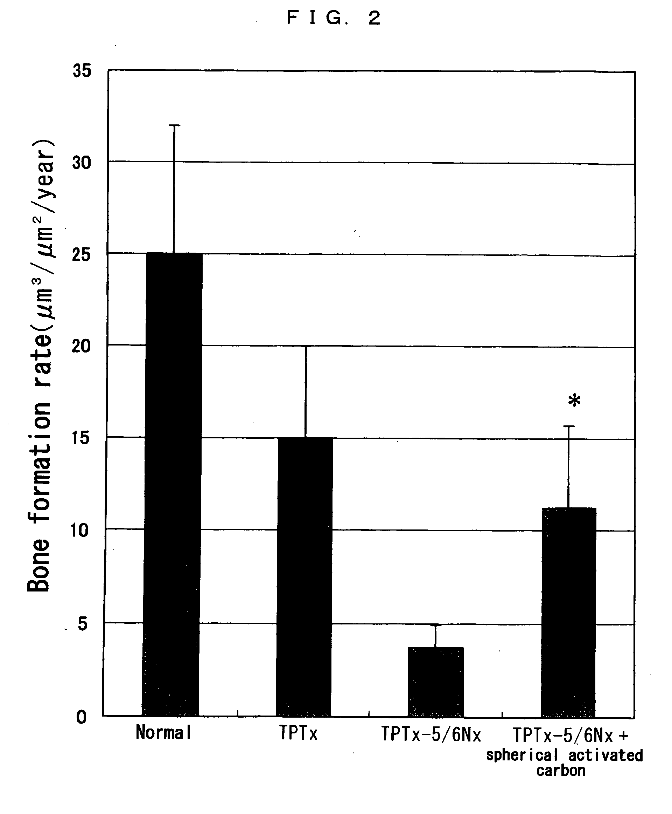

The result is shown in Figure 2. The symbol "*" in Figure 2 means p<0.05 with respect to the TPTx-5/6Nx rat. As shown in Figure 2, the bone formation rate in the administered group was improved in comparison with the control group, and the symptoms were alleviated.

INDUSTRIAL APPLICABILITY

According to the medicament of the present invention, various diseases caused by a low-turnover bone, such as renal osteodystrophy (particularly renal osteodystrophy caused by a low-turnover bone) or adynamic bone disease (preferably adynamic bone disease in conservative chronic kidney disease, or renal osteodystrophy in a patient under treatment with dialysis), can be treated or prevented.

Although the present invention has been described with reference to specific embodiments, various changes and modifications obvious to those skilled in the art are possible without departing from the scope of the appended claims.

**Claims**

1. An agent for treating or preventing a low-turnover bone disease, comprising as an active ingredient a spherical activated carbon.

2. The agent according to claim 1, wherein the low-turnover bone disease is renal osteodystrophy or adynamic bone disease.

3. The agent according to claim 2, wherein the adynamic bone disease is adynamic bone disease in conservative chronic kidney disease or adynamic bone disease in renal osteodystrophy in a patient under treatment with dialysis.

4. The agent according to any one of claims 1 to 3, wherein a particle size of the spherical activated carbon is 0.01 to 2 mm.

5. A pharmaceutical composition for treating or preventing a low-turnover bone disease, comprising a spherical activated carbon and a pharmaceutically or veterinarily acceptable carrier or diluent.

6. The pharmaceutical composition according to claim 5, wherein the low-turnover bone disease is renal osteodystrophy or adynamic bone disease.

7. The pharmaceutical composition according to claim 6, wherein the adynamic bone disease is adynamic bone disease in conservative chronic kidney disease, or adynamic bone disease in renal osteodystrophy in a patient under treatment with dialysis.

8. The pharmaceutical composition according to any one of claims 5 to 6, wherein a particle size of the spherical activated carbon is 0.01 to 2 mm.

9. A method for treating or preventing a low-turnover bone disease, comprising administering to a subject in need thereof a spherical activated carbon in an amount effective thereof.

10. The method according to claim 9, wherein the low-turnover bone disease is renal osteodystrophy or adynamic bone disease.

11. The method according to claim 10, wherein the adynamic bone disease is adynamic bone disease in conservative chronic kidney disease, or adynamic bone disease in renal osteodystrophy in a patient under treatment with dialysis.

12. The method according to any one of claims 9 to 11, wherein a particle size of the spherical activated carbon is 0.01 to 2 mm.

13. Use of a spherical activated carbon in the manufacture of an agent for treating or preventing a low-turnover bone disease or a pharmaceutical composition for treating or preventing a low-turnover bone disease.

14. Use according to claim 13, wherein the low-turnover bone disease is renal osteodystrophy or adynamic bone disease.

15. Use according to claim 14, wherein the adynamic bone disease is adynamic bone disease in conservative chronic kidney disease, or adynamic bone disease in renal osteodystrophy in a patient under treatment with dialysis.

16. Use according to any one of claims 13 to 15, wherein a particle size of the spherical activated carbon is 0.01 to 2 mm.

FIG. 1

F I G. 2

**EP 1 609 475 A1**

## INTERNATIONAL SEARCH REPORT

| | International application No. |
|---|---|
| | PCT/JP2004/004839 |

**A. CLASSIFICATION OF SUBJECT MATTER**
Int.Cl⁷ A61K33/44, A61P19/08, A61P13/12

According to International Patent Classification (IPC) or to both national classification and IPC

**B. FIELDS SEARCHED**

Minimum documentation searched (classification system followed by classification symbols)
Int.Cl⁷ A61K33/44, A61P19/08, A61P13/12

Documentation searched other than minimum documentation to the extent that such documents are included in the fields searched

Electronic data base consulted during the international search (name of data base and, where practicable, search terms used)
CAPlus(STN), REGISTRY(STN), BIOSIS(STN), EMBASE(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| X | JP 11-292771 A (Kureha Chemical Industry Co., Ltd.), 26 October, 1999 (26.10.99), Claims 3, 4; examples (Family: none) | 1-8,13-16 |
| Y | JP 2002-308785 A (Kureha Chemical Industry Co., Ltd.), 23 October, 2002 (23.10.02), Examples & US 2002/0176840 A1 & US 2003/0118581 A1 | 1-8,13-16 |
| Y | Takako IWASAKI et al., "Jin Kino Teika wa Kotsu taisha Kaiten no Teika o Yudo suru", The Japanese Journal of Nephrology, 2002, Vol.44, No.3, page 327 | 1-8,13-16 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 June, 2004 (08.06.04) | 29 June, 2004 (29.06.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

15

**INTERNATIONAL SEARCH REPORT**

International application No.

PCT/JP2004/004839

C (Continuation).   DOCUMENTS CONSIDERED TO BE RELEVANT

| Category* | Citation of document, with indication, where appropriate, of the relevant passages | Relevant to claim No. |
|---|---|---|
| Y | Takako IWASAKI et al., "Jin Kino Teika ga Mukei seikotsu eno Iko o Sokushin suru", Japanese journal of bone metabolism, 2001, Vol.19, No.2, page 140 | 1-8,13-16 |

Form PCT/ISA/210 (continuation of second sheet) (January 2004)

**INTERNATIONAL SEARCH REPORT**

International application No.

PCT/JP2004/004839

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 9-12
   because they relate to subject matter not required to be searched by this Authority, namely:
   Claims 9 to 12 pertain to methods for treatment of the human body by therapy and thus relate to a subject matter which this International Searching Authority is not required, under the provisions of Article 17 (2)(a)(i) of the PCT and Rule of 39.1 (iv) of the Regulations under the PCT, to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**          ☐   The additional search fees were accompanied by the applicant's protest.

☐   No protest accompanied the payment of additional search fees.

Form PCT/ISA/210 (continuation of first sheet (2)) (January 2004)